# EUROPEAN PATENT APPLICATION

(11) **EP 3 682 868 A1**
(43) Date of publication of application: **22.07.2020**
(21) Application number: 19201242.5
(22) Date of filing: 03.10.2019
(51) Int. Cl.: A61K 9/127, A61K 9/51, C12N 15/88

(54) **METHOD OF MANUFACTURING VESICLES BY DELIVERY OF RNA NANOPARTICLES AND VESICLES MANUFACTURED USING THE SAME**

(30) Priority: 16.01.2019 KR 20190005784
(71) Applicant: University Of Seoul Industry Cooperation Foundation, Seoul 130-743 (KR)
(72) Inventor: LEE, Jong-Bum, 138-890 Seoul (KR); KIM, Hye-Jin, 02498 Seoul (KR)
(74) Representative: González López-Menchero, Álvaro Luis

(57) **Abstract**

The present invention relates to a method of manufacturing vesicles by delivery of RNA nanoparticles, in which messenger RNA nanoparticles for target protein expression are delivered to a cell, and vesicles manufactured using the same. A protein is locally over-expressed in the cell to thus be excreted through the vesicles to the outside of the cell, which enables the vesicles containing a target protein to be easily mass-produced. The vesicles containing the target protein is obtained regardless of the cell type. The concentration of the messenger RNA nanoparticles delivered to the cell is adjusted, thus adjusting the manufacturing amount and the manufacturing time of the vesicles. After the surface of the cell is reformed, the messenger RNA nanoparticles are delivered thereto, thus obtaining the vesicles carrying the target protein and having a surface property of a specific function.

## Description

### BACKGROUND OF THE INVENTION

### 1. Field of the Invention

The present invention relates to a method of manufacturing vesicles by delivery of RNA nanoparticles and vesicles manufactured using the same. More particularly, the present invention relates to a method of manufacturing vesicles by delivery of RNA nanoparticles, in which messenger RNA nanoparticles for target protein expression are delivered to a cell, and vesicles manufactured using the same. A protein is locally over-expressed in the cell to thus be excreted through the vesicles to the outside of the cell, which enables the vesicles containing a target protein to be easily mass-produced. The vesicles containing the target protein is obtained regardless of the cell type. The concentration of the messenger RNA nanoparticles delivered to the cell is adjusted, thus adjusting the manufacturing amount and the manufacturing time of the vesicles. After the surface of the cell is reformed, the messenger RNA nanoparticles are delivered thereto, thus obtaining the vesicles carrying the target protein and having a surface property of a specific function. The vesicles carrying not only the target protein but also target DNA or RNA delivered in advance into the cell is obtained without an additional carrying process.

### 2. Description of the Related Art

Extracellular vesicles (EV) is released from cells to the external environment and has a structure surrounded by a phospholipid bilayer. The vesicles is classified into an exosome, a microvesicle, and an apoptotic body according to the generation type, among which the exosome has a size of 50 to 200 nm and the microvesicle has a size ranging from a hundred nanometers to several micrometers. The cell-derived vesicles is naturally secreted from various cells of eukaryotes, such as caryospheres, platelets, endothelial cells, and cancer cells, has a membrane composition that is the same as or similar to that of a secretory cell, and includes the cytoplasm part of a parent cell. Therefore, the cell-derived vesicles includes elements such as a variety of genetic materials, such as miRNA, and proteins, and studies on the manufacture of vesicles to utilize the elements thereof as a cell-signaling-substance-delivery system, a drug delivery system, or an immunotherapeutic agent have been actively carried out.

One of the methods for obtaining cell-derived vesicles is to collect naturally secreted vesicles, which has drawbacks in that the yield is very low and the substances contained therein cannot be adjusted. In order to overcome such drawbacks, a method of producing vesicles containing a target protein by physically and chemically treating transformed cells has been developed, and is described in the following patent documents.

### <Patent Document>

Korean Patent No. 10-1733971 (registered on May 1, 2017) entitled "Manufacturing method of exosome containing target protein, and method of delivering target protein to cytoplasm using exosome manufactured by the manufacturing method"

However, a conventional method of producing vesicles containing a target protein involves physically and chemically treating transformed cells, which complicates processing and lowers the efficiency of vesicles production.

### SUMMARY OF THE INVENTION

Accordingly, the present invention has been made keeping in mind the above problems occurring in the related art, and
an object of the present invention is to provide a method of manufacturing vesicles by delivery of RNA nanoparticles, in which messenger RNA nanoparticles for target protein expression are delivered to a cell. Accordingly, a protein is locally over-expressed in the cell to thus be excreted through the vesicles to the outside of the cell, which enables the vesicles containing a target protein to be easily mass-produced.

Another object of the present invention is to provide a method of manufacturing vesicles by delivery of RNA nanoparticles, in which the vesicles containing a target protein is obtained regardless of the cell type.

Another object of the present invention is to provide a method of manufacturing vesicles by delivery of RNA nanoparticles, in which the concentration of the messenger RNA nanoparticles delivered to a cell is adjusted, thus adjusting the manufacturing amount and the manufacturing time of the vesicles.

Another object of the present invention is to provide a method of manufacturing vesicles by delivery of RNA nanoparticles, in which, since the vesicles that is manufactured has the surface properties of a cell, after the surface of the cell is reformed, messenger RNA nanoparticles are delivered thereto, thus obtaining the vesicles carrying a target protein and having a surface property of a specific function.

Another object of the present invention is to provide a method of manufacturing vesicles by delivery of RNA nanoparticles, in which the vesicles carrying not only a target protein but also target DNA or RNA delivered in advance into a cell is obtained without an additional carrying process.

In order to accomplish the above objects, the present invention is implemented by the embodiments having the following constitutions.

According to an embodiment of the present invention, a method of manufacturing vesicles according to the present invention includes a delivery step of delivering RNA nanoparticles for vesicles production to a cell and a culturing step of culturing the cell to which the RNA nanoparticles for vesicles production are delivered after the delivery step, thus generating the vesicles containing a target protein.

According to another embodiment of the present invention, in the method of manufacturing the vesicles according to the present invention, the RNA nanoparticles for vesicles production include repeated messenger RNA for target protein expression, so that the protein is locally over-expressed in the cell in the culturing step to thus be excreted through the vesicles to the outside of the cell, which enables the vesicles containing the target protein to be produced.

According to another embodiment of the present invention, in the method of manufacturing the vesicles according to the present invention, the amount of the RNA nanoparticles for vesicles production delivered to the cell in a delivery step is adjusted, thus adjusting the generation amount and the manufacturing time of the vesicles.

According to another embodiment of the present invention, in the method of manufacturing the vesicles according to the present invention, the RNA nanoparticles for vesicles production have a spherical shape and are 50 to 200 nm in diameter.

According to another embodiment of the present invention, the method of manufacturing the vesicles according to the present invention further includes a surface-reforming step of reforming a surface of the cell before a delivery step. Since the vesicles generated during the culturing step has the surface properties of the cell, when the cell having the surface reformed during the surface-reforming step is used in the delivery step, the vesicles carrying the target protein and having a surface property of a specific function is produced in the culturing step.

According to another embodiment of the present invention, in the method of manufacturing the vesicles according to the present invention, a delivery step includes a process using a liposome or a positively charged polymer, a delivery process using the liposome includes adding the RNA nanoparticles to a lipid-based-complex-forming solution and then forming a complex in order to treat the cell, and a process using the positively charged polymer includes dispensing the cell on a culture dish, adding a growth medium thereto to culture the cell for a predetermined time, and adding a complex obtained by mixing the RNA nanoparticles and the positively charged polymer to the growth medium.

According to another embodiment of the present invention, the method of manufacturing the vesicles according to the present invention further includes a separation step of separating the vesicles generated in the culturing step.

According to another embodiment of the present invention, in the method of manufacturing the vesicles according to the present invention, in the separation step, the vesicles containing a target protein is separated from a cell culture medium using centrifugation.

According to the present invention, the following effects may be obtained through these embodiments.

The present invention has an effect of making it easy to mass-produce vesicles containing a target protein by delivering messenger RNA nanoparticles for target protein expression to a cell, so that the protein is locally over-expressed in the cell to thus be excreted through the vesicles to the outside of the cell.

Further, the present invention has an effect of obtaining vesicles containing a target protein regardless of the cell type.

Further, the present invention has an effect of adjusting the manufacturing amount and a manufacturing time of vesicles by adjusting the concentration of the messenger RNA nanoparticles delivered to a cell.

Further, the present invention has an effect of obtaining vesicles carrying a target protein and having a surface property of a specific function by reforming the surface of a cell and then delivering messenger RNA nanoparticles thereto because the vesicles that is manufactured has the surface properties of the cell.

Further, the present invention has an effect of obtaining vesicles carrying not only a target protein but also target DNA or RNA delivered in advance into a cell without an additional carrying process.

### BRIEF DESCRIPTION OF THE DRAWINGS

The above and other objects, features and advantages of the present invention will be more clearly understood from the following detailed description taken in conjunction with the accompanying drawings, in which:
FIG. 1 is a view showing an atomic force microscopic image, a scanning electron microscopic image, and the result of dynamic light scattering analysis of messenger RNA nanoparticles;
FIG. 2 is a view showing fluorescence microscopic images and cytometry analysis results for confirming the formation of vesicles by delivering messenger RNA nanoparticles for GFP expression to a PC-3 cell;
FIG. 3 shows scanning electron microscopic images for confirming the formation of vesicles by delivering messenger RNA nanoparticles for GFP expression to a PC-3 cell;
FIG. 4 is a view showing fluorescence microscopic images and ImageJ software analysis results for confirming the formation of vesicles by delivering messenger RNA nanoparticles for GFP expression to a PC-3 cell;
FIG. 5 shows fluorescence microscopic images for confirming the formation of vesicles over time by delivering messenger RNA nanoparticles for GFP expression to a PC-3 cell;
FIG. 6 shows fluorescence microscopic images for confirming the formation of vesicles over time by delivering messenger RNA nanoparticles for GFP expression to a HeLa cell;
FIG. 7 shows fluorescence microscopic images of vesicles formed by delivering messenger RNA nanoparticles for GFP expression to a HeLa cell;
FIG. 8 shows fluorescence microscopic images for confirming that vesicles, which is formed by delivering messenger RNA nanoparticles for GFP expression to a HeLa cell, is derived from the HeLa cell;
FIG. 9 shows scanning electron microscopic images for confirming that vesicles, which is formed by delivering messenger RNA nanoparticles for GFP expression to a HeLa cell, is derived from the HeLa cell;
FIG. 10 is a view showing fluorescence microscopic images and cytometry analysis results for confirming the formation of vesicles by delivering messenger RNA nanoparticles for RFP expression to a HeLa cell;
FIG. 11 shows fluorescence microscopic images for confirming the formation of vesicles over time by delivering messenger RNA nanoparticles for RFP expression to a HeLa cell;
FIG. 12 is a view showing scanning electron microscopic images and SEM-based EDX analysis results for confirming the formation of vesicles by delivering messenger RNA nanoparticles for GFP expression to a MDA-MB-231 cell;
FIG. 13 shows scanning electron microscopic images for confirming the formation of vesicles by delivering messenger RNA nanoparticles to an HDF cell;
FIG. 14 shows fluorescence microscopic images for confirming the formation of vesicles by delivering messenger RNA nanoparticles to an HDF cell;
FIG. 15 shows fluorescence microscopic images for confirming that the amount of vesicles production is capable of being adjusted depending on the concentration of messenger RNA nanoparticles for RFP expression in treatment;
FIG. 16 shows scanning electron microscopic images for confirming that the amount of vesicles production is capable of being adjusted depending on the concentration of messenger RNA nanoparticles for RFP expression in treatment;
FIG. 17 is a view showing EDX analysis results for confirming that the amount of vesicles production is capable of being adjusted depending on the concentration of messenger RNA nanoparticles for RFP expression in treatment;
FIG. 18 shows scanning electron microscopic images for confirming that the amount of vesicles production is capable of being adjusted depending on the concentration of messenger RNA nanoparticles for GFP expression in treatment;
FIG. 19 shows fluorescence microscopic images for confirming that functions are capable of being provided to vesicles by reforming the surface of a cell; and
FIG. 20 shows fluorescence microscopic images for confirming that a cell-derived vesicles is capable of being used as a target protein delivery system.

### DESCRIPTION OF THE PREFERRED EMBODIMENTS

Hereinafter, a method of manufacturing vesicles by delivery of RNA nanoparticles and vesicles manufactured using the same according to the present invention will be described in detail with reference to the drawings. Unless defined otherwise, all terms used in the present specification have the same meanings as those commonly understood by one of ordinary skill in the art to which the present invention belongs, and in the case of conflict with the meanings of the terms used in the present specification, the terms follow the definition used in the present specification. Further, the detailed description of known functions and configurations that may unnecessarily obscure the subject matter of the present invention will be omitted. Throughout the specification, when a component is referred to as "including" an element, it is understood that the component may include other elements as well, without departing from the other elements unless specifically stated otherwise.

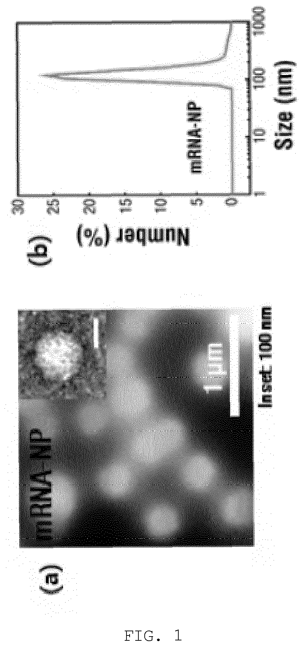

A method of manufacturing vesicles by delivery of RNA nanoparticles according to an embodiment of the present invention will be described with reference to FIGS. 1 to 20. The method of manufacturing the vesicles includes a particle-manufacturing step of manufacturing RNA nanoparticles for vesicles production, a delivery step of delivering the RNA nanoparticles for vesicles production, which are manufactured in the particle-manufacturing step, to a cell, a culturing step of culturing the cell to which the RNA nanoparticles for vesicles production are delivered after the delivery step, thus generating the vesicles containing a target protein, and a separation step of separating the vesicles generated in the culturing step.

The particle-manufacturing step is a step of manufacturing RNA nanoparticles for vesicles production, and messenger RNA nanoparticles including repeated messenger RNA for target protein expression are manufactured. The RNA nanoparticles for vesicles production have a predetermined shape and size, and preferably have a spherical shape overall and a diameter of 50 to 200 nm. The single-stranded messenger RNAs are twisted and tangled with each other to form the messenger RNA nanoparticles, and the messenger RNA nanoparticles include only biomaterials, and thus are not toxic to the body. Stable in the interior environment of the body, the messenger RNA nanoparticles may release mRNA continuously over a long period of time.

The particle-manufacturing step includes a pDNA-generating step of generating circular double-stranded plasmid DNA, which includes a base sequence complementary to a messenger RNA base sequence for target protein expression, a ribosome-binding base sequence essential for translation of a protein from RNA, and a promoter base sequence for a T7 RNA polymerase and from which a termination base sequence is removed, and a particle-forming step of incubating a reaction solution containing the plasmid DNA and the RNA polymerase at a predetermined temperature for a predetermined time, so that the plasmid DNA was subjected to rolling circle transcription (RCT) using the RNA polymerase, whereby long single-stranded messenger RNAs including the repeated messenger RNA base sequence for target protein expression are generated, and the generated single-stranded messenger RNAs are twisted and tangled with each other to form messenger RNA nanoparticles through self-assembly. Since the termination base sequence is removed from the plasmid DNA, the RNA may be continuously produced by the RNA polymerase on the circular plasmid DNA in the particle-forming step, so that the mRNA base sequence may be repeatedly loaded on the single-stranded RNA.

The delivery step is a step of delivering the RNA nanoparticles for vesicles production manufactured in the particle-manufacturing step to a cell. For example, the RNA nanoparticles for vesicles production may be delivered to the cell using a liposome or a positively charged polymer. The amount of the RNA nanoparticles for vesicles production delivered to the cell in the delivery step may be adjusted, thus adjusting the manufacturing amount and the manufacturing time of the vesicles.

The delivery process using the liposome may include mixing the RNA nanoparticles and a lipid-based-complex-forming solution (Stemfect RNA Transfection Kit, Stemgent) to generate a complex and then adding the complex to a cell culture medium. The process using the positively charged polymer may include dispensing a cell on a culture dish, adding a growth medium thereto to perform culturing for a predetermined time, mixing the RNA nanoparticles with a positively-charged-polymer-phosphorus-based transfection reagent (TransIT-X2 Dynamic Delivery System, Mirus) to generate a complex, and adding the complex to a cell culture medium.

The culturing step is a step of culturing the cell, to which the RNA nanoparticles for vesicles production are delivered, at a predetermined temperature for a predetermined time after the delivery step, thus generating the vesicles containing the target protein. In the culturing step, the messenger RNA nanoparticles for target protein expression may be delivered to the cell, so that the protein is locally over-expressed in the cell to thus be excreted through the vesicles to the outside of the cell, which enables the vesicles containing the target protein to be easily mass-produced. Further, in the method of manufacturing the vesicles, the RNA nanoparticles for vesicles production may be manufactured to express various target proteins through the particle-manufacturing step. In the delivery step, the RNA nanoparticles for vesicles production are capable of being delivered to various cells, thus obtaining vesicles containing the target protein regardless of the cell type.

The separating step is a step of separating the vesicles generated in the culturing step. For example, the vesicles containing the target protein may be obtained from the cell culture medium using a process such as centrifugation.

In another embodiment of the present invention, a surface-reforming step of reforming the surface of the cell using metabolic engineering may be further included between the particle-manufacturing step and the delivery step, which imparts a new function such as target recognition to the surface of the vesicles. Since the vesicles manufactured by the method of manufacturing the vesicles has the surface properties of the cell, after the surface of the cell is reformed, the RNA nanoparticles for vesicles production may be delivered to obtain vesicles carrying a target protein and having a surface property of a specific function. Further, in the case of a microvesicle, budding occurs directly from the cytoplasm, unlike other extracellular vesicles. As a result, the RNA and DNA contained in the cytoplasm may be naturally carried while expressing the target protein and carrying the corresponding protein in the microvesicle. Accordingly, when the RNA and DNA are contained together in the manufacture of the RNA nanoparticles for vesicles production, it is possible to generate a microvesicle containing all of a target protein, DNA, and RNA.

Another embodiment of the present invention includes vesicles containing a target protein manufactured using the method of manufacturing the vesicles.

Hereinafter, the present invention will be described in more detail with reference to Examples. However, these are only for the purpose of illustrating the present invention in more detail, and the scope of the present invention is not limited thereto.

### <Example 1> Manufacture of messenger RNA nanoparticles for vesicles production

1. Plasmid DNA
(CCCGTGTAAAACGACGGCCAGTTTATCTAGTCAGCTTGATTCTAGCTGATCGTGGACCGGAAG
GTGAGCCAGTGAGTTGATTGCAGTCCAGTTACGCTGGAGTCTGAGGCTCGTCCTGAATGATATG
CGACCGCCGGAGGGTTGCGTTTGAGACGGGCGACAGATCGACACTGCTCGATCCGCTCGCACC-
TAATACGACTCACTATAGG (Sequence number: 1)-GAT-GCCACCATGG
(Sequence number: 2)-

ATGGTGAGCAAGGGCGAGGAGCTGTTCACCGGGGTGGTGCCCATCCTGGTCGAGCTGG
ACGGCGACGTAAACGGCCACAAGTTCAGCGTGTCCGGCGAGGGCGAGGGCGATGCCACCTACGG
CAAGCTGACCCTGAAGTTCATCTGCACCACCGGCAAGCTGCCCGTGCCCTGGCCCACCCTCGTG
ACCACCCTGACCTACGGCGTGCAGTGCTTCAGCCGCTACCCCGACCACATGAAGCAGCACGACT
TCTTCAAGTCCGCCATGCCCGAAGGCTACGTCCAGGAGCGCACCATCTTCTTCAAGGACGACGG
CAACTACAAGACCCGCGCCGAGGTGAAGTTCGAGGGCGACACCCTGGTGAACCGCATCGAGCTG
AAGGGCATCGACTTCAAGGAGGACGGCAACATCCTGGGGCACAAGCTGGAGTACAACTACAACA
GCCACAACGTCTATATCATGGCCGACAAGCAGAAGAACGGCATCAAGGTGAACTTCAAGATCCG
CCACAACATCGAGGACGGCAGCGTGCAGCTCGCCGACCACTACCAGCAGAACACCCCCATCGGC
GACGGCCCCGTGCTGCTGCCCGACAACCACTACCTGAGCACCCAGTCCGCCCTGAGCAAAGACC
CCAACGAGAAGCGCGATCACATGGTCCTGCTGGAGTTCGTGACCGCCGCCGGGATCACTCTCGG
CATGGACGAGCTGTACAAGTAA (Sequence number: 3)

GGATCGACGAGAGCAGCGCGACTGGATCAGTTCTGGACGAGCGAGCTGTCGTCCGACC CGTGATCTTACGGCATTATACGTATGATCGGTCCACGATCAGCTAGATTATCTAGTCAGCTTGA TGTCATAGCTGTTTCCTGAGGCTCAATACTGACCATTTAAATCATACCTGACCTCCATAGCAGA AAGTCAAAAGCCTCCGACCGGAGGCTTTTGACTTGATCGGCACGTAAGAGGTTCCAACTTTCAC CATAATGAAATAAGATCACTACCGGGCGTATTTTTTGAGTTATCGAGATTTTCAGGAGCTAAGG AAGCTAAAATGAGTATTCAACATTTCCGTGTCGCCCTTATTCCCTTTTTTGCGGCATTTTGCCT TCCTGTTTTTGCTCACCCAGAAACGCTGGTGAAAGTAAAAGATGCTGAAGATCAGTTGGGTGCA CGAGTGGGTTACATCGAACTGGATCTCAACAGCGGTAAGATCCTTGAGAGTTTACGCCCCGAAG AACGTTTTCCAATGATGAGCACTTTTAAAGTTCTGCTATGTGGCGCGGTATTATCCCGTATTGA CGCCGGGCAAGAGCAACTCGGTCGCCGCATACACTATTCTCAGAATGACTTGGTTGAGTACTCA CCAGTCACAGAAAAGCATCTCACGGATGGCATGACAGTAAGAGAATTATGCAGTGCTGCCATAA CCATGAGTGATAACACTGCGGCCAACTTACTTCTGGCAACGATCGGAGGACCGAAGGAGCTAAC CGCTTTTTTGCACAACATGGGGGATCATGTAACTCGCCTTGATCGTTGGGAACCGGAGCTGAAT GAAGCCATACCAAACGACGAGCGTGACACCACGATGCCTGTAGCAATGGCAACAACGTTGCGCA AACTATTAACTGGCGAACTACTTACTCTAGCTTCCCGGCAACAATTAATAGACTGGATGGAGGC GGATAAAGTTGCAGGATCACTTCTGCGCTCGGCCCTCCCGGCTGGCTGGTTTATTGCTGATAAA TCTGGAGCCGGTGAGCGTGGGTCTCGCGGTATCATTGCAGCACTGGGGCCAGATGGTAAGCCCT CCCGCATCGTAGTTATCTACACGACGGGGAGTCAGGCAACTATGGATGAACGAAATAGACAGAT CGCTGAGATAGGTGCCTCACTGATTAAGCATTGGTAATGAGGGCCCAAATGTAATCACCTGGCT CACCTTCGGGTGGGCCTTTCTTGAGGACCTAAATGTAATCACCTGGCTCACCTTCGGGTGGGCC TTTCTGCGTTGCTGGCGTTTTTCCATAGGCTCCGCCCCCCTGACGAGCATCACAAAAATCGATG CTCAAGTCAGAGGTGGCGAAACCCGACAGGACTATAAAGATACCAGGCGTTTCCCCCTGGAAGC TCCCTCGTGCGCTCTCCTGTTCCGACCCTGCCGCTTACCGGATACCTGTCCGCCTTTCTCCCTT CGGGAAGCGTGGCGCTTTCTCATAGCTCACGCTGTAGGTATCTCAGTTCGGTGTAGGTCGTTCG CTCCAAGCTGGGCTGTGTGCACGAACCCCCCGTTCAGCCCGACCGCTGCGCCTTATCCGGTAAC TATCGTCTTGAGTCCAACCCGGTAAGACACGACTTATCGCCACTGGCAGCAGCCACTGGTAACA GGATTAGCAGAGCGAGGTATGTAGGCGGTGCTACAGAGTTCTTGAAGTGGTGGCCTAACTACGG CTACACTAGAAGAACAGTATTTGGTATCTGCGCTCTGCTGAAGCCAGTTACCTCGGAAAAAGAG TTGGTAGCTCTTGATCCGGCAAACAAACCACCGCTGGTAGCGGTGGTTTTTTTGTTTGCAAGCA GCAGATTACGCGCAGAAAAAAAGGATCTCAAGAAGATCCTTTGATTTTCTACCGAAGAAAGGCC CA) was designed, which included a promoter base sequence (Sequence number: 1) for T7 RNA polymerase, a ribosome-binding base sequence (Kozak base sequence) (Sequence number: 2) essential for translation of a protein from RNA, and a messenger RNA base sequence (Sequence number: 3) for expression of a green fluorescent protein (GFP), which was a target protein and from which a termination base sequence was removed. 1 nM plasmid DNA, 1 mM ribonucleotide solution mix (New England Biolabs), a reaction buffer (8 mM Tris-HCl, 0.4 mM spermidine, 1.2 mM MgCl₂, and 2 mM dithiothreitol), and T7 RNA polymerase (50 units per 1 ml, England BioLabs) were added to a tube and then mixed. The resultant tube was put into an incubator, followed by reaction at 37°C for 20 hours, thus manufacturing messenger RNA nanoparticles for vesicles production (mRNA-NP).
2. Messenger RNA nanoparticles for vesicles production (mRNA-NP) were manufactured under the same conditions as in item 1 of Example 1, except that plasmid DNA (TCGCGCGTTTCGGTGATGACGGTGAAAACCTCTGACACATGCAGCTCCCGGAGACGGTCACAG CTTGTCTGTAAGCGGATGCCGGGAGCAGACAAGCCCGTCAGGGCGCGTCAGCGGGTGTTGGCGG GTGTCGGGGCTGGCTTAACTATGCGGCATCAGAGCAGATTGTACTGAGAGTGCACCAAATGCGG TGTGAAATACCGCACAGATGCGTAAGGAGAAAATACCGCATCAGGCGCCATTCGCCATTCAGGC TGCGCAACTGTTGGGAAGGGCGATCGGTGCGGGCCTCATCGCTATTACGCCAGCTGGCGAAAGG GGGATGTGCTGCAAGGCGATTAAGTTGGGTAACGCCAGGGTTTTCCCAGTCACGACGTTGTAAA ACGACGGCCAGTGCAACGCGATGACGATGGATAGCGATTCATCGATGAGCTGACCCGATCGCCG CCGCCGGAGGGTTGCGTTTGAGACGGGCGACAGATGAGGCTCGTTTAGTGAACCGTCAGATCGC CTGGAGACGCCATCCACGCTGTTTTGACCTCCATAGAAGACACCGGGACCGATCCAGCCTCCGG ACTCTAGAGGATCGAACCCTTTTGGACCCTCGTACAGAAGC-TAATACGACTCACTATAGG (Sequence number: 1)- 
GAAATAAGAGAGAAAAGAAGAGTAAGAAGAAATATAAGAGCCACCGCCCCTCTCCCTC
CCCCCCCCCTAACGTTACTGGCCGAAGCCGCTTGGAATAAGGCCGGTGTGCGTTTGTCTATATG
TTATTTTCCACCATATTGCCGTCTTTTGGCAATGTGAGGGCCCGGAAACCTGGCCCTGTCTTCT
TGACGAGCATTCCTAGGGGTCTTTCCCCTCTCGCCAAAGGAATGCAAGGTCTGTTGAATGTCGT
GAAGGAAGCAGTTCCTCTGGAAGCTTCTTGAAGACAAACAACGTCTGTAGCGACCCTTTGCAGG
CAGCGGAACCCCCCACCTGGCAACAGGTGCCTCTGCGGCCAAAAGCCACGTGTATAAGATACAC
CTGCAAAGGCGGCACAACCCCAGTGCCACGTTGTGAGTTGGATAGTTGTGGAAAGAGTCAAATG
 
GCTCTCCTCAAGCGTATTCAACAAGGGGCTGAAGGATGCCCAGAAGGTACCCCATTGTATGGGA
TCTGATCTGGGGCCTCGGTGCACATGCTTTACATGTGTTTAGTCGAGGTTAAAAAACGTCTAG
GCCCCCCGAACCACGGGGACGTGGTTTTCCTTTGAAAAACACGATGATAA (Sequence
number: 4)-T-
 
ATGGATAGCACTGAGAACGTCATCAAGCCCTTCATGCGCTTCAAGGTGCACATGGAGG
GCTCCGTGAACGGCCACGAGTTCGAGATCGAGGGCGAGGGCGAGGGCAAGCCCTACGAGGGCAC
CCAGACCGCCAAGCTGCAGGTGACCAAGGGCGGCCCCCTGCCCTTCGCCTGGGACATCCTGTCC
CCCCAGTTCCAGTACGGCTCCAAGGTGTACGTGAAGCACCCCGCCGACATCCCCGACTACAAGA
AGCTGTCCTTCCCCGAGGGCTTCAAGTGGGAGCGCGTGATGAACTTCGAGGACGGCGGCGTGGT
GACCGTGACCCAGGACTCCTCCCTGCAGGACGGCACCTTCATCTACCACGTGAAGTTCATCGGC
GTGAACTTCCCCTCCGACGGCCCCGTAATGCAGAAGAAGACTCTGGGCTGGGAGCCCTCCACCG
AGCGCCTGTACCCCCGCGACGGCGTGCTGAAGGGCGAGATCCACAAGGCGCTGAAGCTGAAGGG
CGGCGGCCACTACCTGGTGGAGTTCAAGTCAATCTACATGGCCAAGAAGCCCGTGAAGCTGCCC
GGCTACTACTACGTGGACTCCAAGCTGGACATCACCTCCCACAACGAGGACTACACCGTGGTGG
AGCAGTACGAGCGCGCCGAGGCCCGCCACCACCTGTTCCAGTAG (Sequence number:
5)-
 GCTGCCTTCTGCGGGGCTTGCCTTCTGGCCATGCCCTTCTTCTCTCCCTTGCACCTGT ACCTCTTGGTCTTTGAATAAAGCCTGAGTAGGAAGTGAGGGTCTAGAACTAGTGTCGACGCAAA TCAGTTCTGGACCAGCGAGCTGTGCTGCGACTCGTGGCGTAATCATGGTCATAGCTGTTTCCTG TGTGAAATTGTTATCCGCTCACAATTCCACACAACATACGAGCCGGAAGCATAAAGTGTAAAGC CTGGGGTGCCTAATGAGTGAGCTAACTCACATTAATTGCGTTGCGCTCACTGCCCGCTTTCCAG TCGGGAAACCTGTCGTGCCAGCTGCATTAATGAATCGGCCAACGCGCGGGGAGAGGCGGTTTGC GTATTGGGCGCTCTTCCGCTTCCTCGCTCACTGACTCGCTGCGCTCGGTCGTTCGGCTGCGGCG AGCGGTATCAGCTCACTCAAAGGCGGTAATACGGTTATCCACAGAATCAGGGGATAACGCAGGA AAGAACATGTGAGCAAAAGGCCAGCAAAAGGCCAGGAACCGTAAAAAGGCCGCGTTGCTGGCGT TTTTCCATAGGCTCCGCCCCCCTGACGAGCATCACAAAAATCGACGCTCAAGTCAGAGGTGGCG AAACCCGACAGGACTATAAAGATACCAGGCGTTTCCCCCTGGAAGCTCCCTCGTGCGCTCTCCT GTTCCGACCCTGTCGCTTACCGGATACCTGTCCGCCTTTCTCCCTTCGGGAAGCGTGGCGCTTT CTCATAGCTCACGCTGTAGGTATCTCAGTTCGGTGTAGGTCGTTCGCTCCAAGCTGGGCTGTGT GCACGAACCCCCCGTTCAGCCCGACCGCTGCGCCTTATCCGGTAACTATCGTCTTGAGTCCAAC CCGGTAAGACACGACTTATCGCCACTGGCAGCAGCCACTGGTAACAGGATTAGCAGAGCGAGGT ATGTAGGCGGTGCTACAGAGTTCTTGAAGTGGTGGCCTAACTACGGCTACACTAGAAGAACAGT ATTTGGTATCTGCGCTCTGCTGAAGCCAGTTACCTTCGGAAAAAGAGTTGGTAGCTCTTGATCC GGCAAACAAACCACCGCTGGTAGCGGTGGTTTTTTTGTTTGCAAGCAGCAGATTACGCGCAGAA AAAAAGGATCTCAAGAAGATCCTTTGATCTTTTCTACGGGGTCTGACGCTCAGTGGAACGAAAA CTCACGTTAAGGGATTTTGGTCATGAGATTATCAAAAAGGATCTTCACCTAGATCCTTTTAAAT TAAAAATGAAGTTTTAAATCAATCTAAAGTATATATGAGTAAACTTGGTCTGACAGTTACCAAT GCTTAATCAGTGAGGCACCTATCTCAGCGATCTGTCTATTTCGTTCATCCATAGTTGCCTGACT CCCCGTCGTGTAGATAACTACGATACGGGAGGGCTTACCATCTGGCCCCAGTGCTGCAATGATA CCGCGAGACCCACGCTCACCGGCTCCAGATTTATCAGCAATAAACCAGCCAGCCGGAAGGGCCG AGCGCAGAAGTGGTCCTGCAACTTTATCCGCCTCCATCCAGTCTATTAATTGTTGCCGGGAAGC TAGAGTAAGTAGTTCGCCAGTTAATAGTTTGCGCAACGTTGTTGCCATTGCTACAGGCATCGTG GTGTCACGCTCGTCGTTTGGTATGGCTTCATTCAGCTCCGGTTCCCAACGATCAAGGCGAGTTA CATGATCCCCCATGTTGTGCAAAAAAGCGGTTAGCTCCTTCGGTCCTCCGATCGTTGTCAGAAG TAAGTTGGCCGCAGTGTTATCACTCATGGTTATGGCAGCACTGCATAATTCTCTTACTGTCATG CCATCCGTAAGATGCTTTTCTGTGACTGGTGAGTACTCAACCAAGTCATTCTGAGAATAGTGTA TGCGGCGACCGAGTTGCTCTTGCCCGGCGTCAATACGGGATAATACCGCGCCACATAGCAGAAC TTTAAAAGTGCTCATCATTGGAAAACGTTCTTCGGGGCGAAAACTCTCAAGGATCTTACCGCTG TTGAGATCCAGTTCGATGTAACCCACTCGTGCACCCAACTGATCTTCAGCATCTTTTACTTTCA CCAGCGTTTCTGGGTGAGCAAAAACAGGAAGGCAAAATGCCGCAAAAAAGGGAATAAGGGCGAC ACGGAAATGTTGAATACTCATACTCTACCTTTTTCAATATTATTGAAGCATTTATCAGGGTTAT TGTCTCATGAGCGGATACATATTTGAATGTATTTAGAAAAATAAACAAATAGGGGTTCCGCGCA CATTTCCCCGAAAAGTGCCACCTGACGTCTAAGAAACCATTATTATCATGACATTAACCTATAA AAATAGGCGTATCACGAGGCCCTTTCGTC), which included a promoter base sequence (Sequence number: 1) for T7 RNA polymerase, a ribosome-binding base sequence (IRES base sequence) (Sequence number: 4) essential for translation of a protein from RNA, and a messenger RNA base sequence (Sequence number: 5) for expression of a red fluorescent protein (RFP(DsRed-Express2)), was used. The green fluorescent protein and the red fluorescent protein were selected to easily confirm the protein that was generated.

### <Example 2> Confirmation of size, shape, and distribution of messenger RNA nanoparticles for vesicles production

FIG. 1(a) shows the result of measurement of the messenger RNA nanoparticles manufactured in item 1 of Example 1 using an atomic force microscope and a transmission electron microscope (inside). FIG. 1(b) shows the results of analysis using dynamic light scattering (DLS). From FIG. 1, nanoparticles having a spherical shape and a diameter of about 100 nm can be confirmed.

### <Example 3> Confirmation of mass-production of vesicles containing target protein by delivery of messenger RNA nanoparticles

### 1. Production of vesicles by delivery of messenger RNA nanoparticles for GFP expression to PC-3 cell

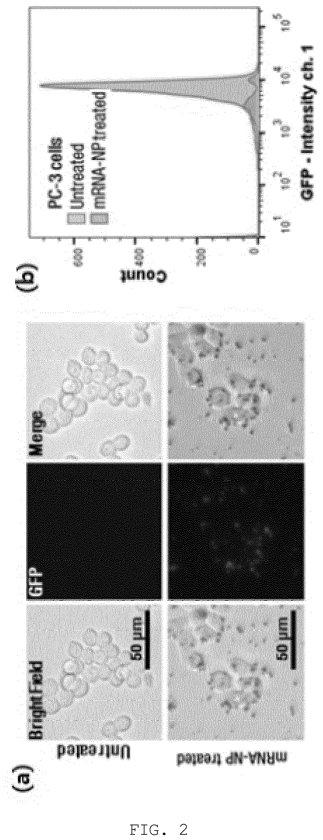

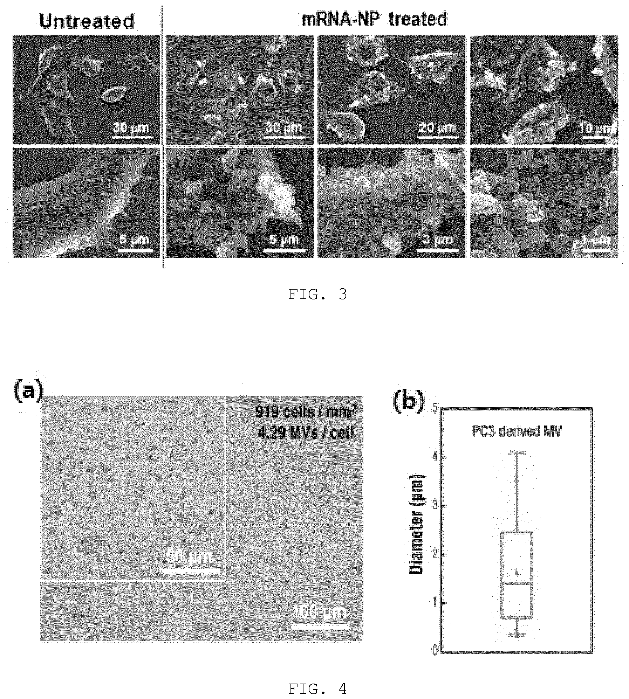

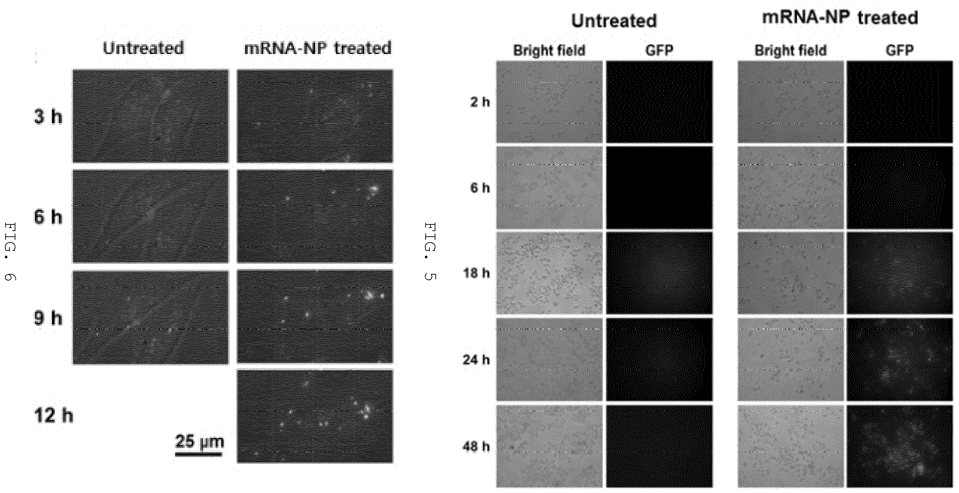

(1) The messenger RNA nanoparticles manufactured in item 1 of Example 1 were diluted in OPTI-MEM I and then mixed with a transfection reagent (TransIT-X2 Dynamic Delivery System) to form a complex (the ratio of messenger RNA nanoparticles: reagent: OPTI MEM I was 7 µg: 15 µl: 50 µl). The complex was added to a PC-3 cell culture medium (20000 cells per cm² were dispensed), followed by reaction (treatment with 1.5 µg of the complex per 1 ml of the cell culture medium). FIG. 2(a) shows the result of measurement using a fluorescence microscope 24 hours after the start of the above reaction, FIG. 2(b) shows the result of confirming GFP expression in cells by cytometry, and FIG. 3 shows the result of measurement using a scanning electron microscope. FIG. 4(a) shows the result of measurement using a fluorescence microscope and the result of analysis of fluorescence microscopic images using ImageJ software, and FIG. 4(b) is a view obtained by plotting the size distributions of the vesicles of the ImageJ software analysis results. Further, FIG. 5 shows the results of measurement using a fluorescence microscope 2, 6, 18, 24, and 48 hours after the start of the above reaction.
(2) From FIG. 2 (a), it can be seen that a large amount of micro-sized vesicles (including GFP) is generated from mRNA-NP-treated cells treated with the messenger RNA nanoparticles for GFP expression, whereas the vesicles is not confirmed in untreated cells. From FIG. 2(b), it can be seen that a large amount of GFP was expressed in the cells treated with the messenger RNA nanoparticles through the fact that the intensity of green fluorescence was significantly higher in the cells treated with the messenger RNA nanoparticles for GFP expression than in the untreated cells. Referring to FIG. 3, the untreated cells have a relatively smooth surface, but in the case of cells whose proteins are expressed by the delivery of the messenger RNA nanoparticles, a large amount of vesicles of several hundred nanometers to several micrometers in size was observed on the cell surface. From FIG. 4(a), it can be seen that at least 4 or more microvesicles (MV) are secreted per cell and that the microvesicles exhibit strong green fluorescence, which means that the microvesicles contain a large amount of GFP proteins. From FIG. 4(b), it can be seen that the microvesicles have an average size of several hundred nanometers to 2 micrometers. From FIG. 5, it can be confirmed that the GFP is relatively strongly expressed from the time point after 18 hours and then the expression of the GFP and the secretion of the vesicles are increased over time. Accordingly, it can be seen that the excessive amount of the protein expressed by the delivery of the messenger RNA nanoparticles for GFP expression has a great influence on the secretion of vesicles from PC-3 cells.

### 2. Production of vesicles by delivery of messenger RNA nanoparticles for GFP expression to HeLa cell

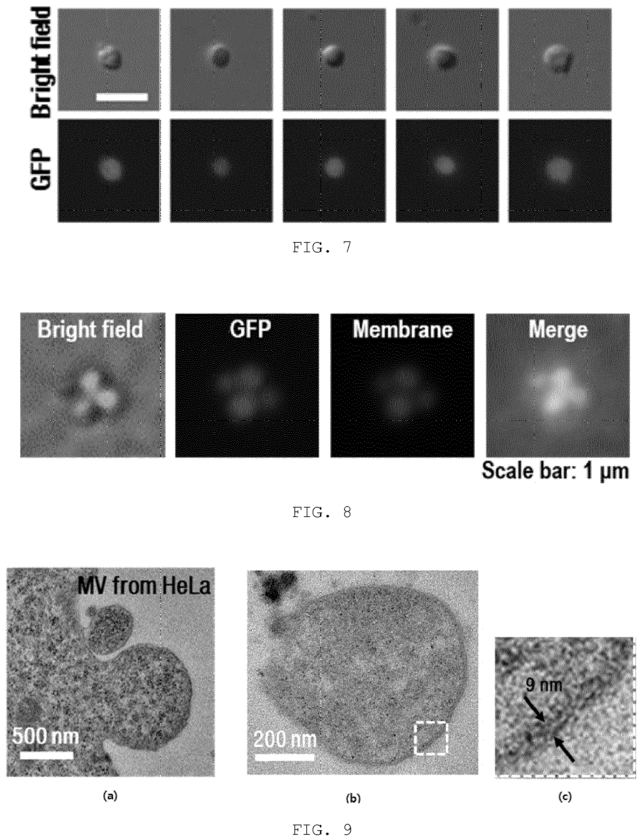

(1) The messenger RNA nanoparticles manufactured in item 1 of Example 1 were diluted in OPTI-MEM I and then mixed with a transfection reagent (TransIT-X2 Dynamic Delivery System) to form a complex (the ratio of messenger RNA nanoparticles: reagent: OPTI MEM I was 7 µg: 15 µl: 50 µl). The complex was added to a HeLa cell culture medium (20000 cells per cm² were dispensed), followed by reaction (treatment with 1.5 µg of the complex per 1 ml of the cell culture medium). FIG. 6 shows the results of measurement using a fluorescence microscope 3, 6, 7, 9, and 12 hours after the start of the above reaction. A plurality of endoplasmic reticula obtained through centrifugation 24 hours after the start of the above reaction was photographed using a fluorescence microscope, and is shown in FIG. 7 (Inset scale bar = 5 µm).
(2) The experiment was performed in the same manner as in sub-item (1) of item 2 of Example 3, except that the HeLa cells stained with CellVue Claret, which was a reagent for staining a cell membrane using a red fluorescent substance, were used. After 24 hours, the vesicles obtained by centrifugation was measured using a fluorescence microscope, and is shown in FIG. 8. Further, the experiment was performed in the same manner as in sub-item (1) of item 2 of Example 3, except that the HeLa cells stained with osmium tetroxide were used, so that the electron density of a phospholipid bilayer portion was increased and thus the phospholipid bilayer could be observed in the image of the transmission electron microscope. After 24 hours, measurement was performed using a transmission electron microscope, and the result is shown in FIG. 9 (FIG. 9(a) shows vesicles that is being secreted from a cell, FIG. 9(b) shows the secreted vesicles, and FIG. 9(c) shows an image obtained by enlarging the dotted-line portion of FIG. 9(b)).
(3) From FIG. 6, it can be confirmed that the expression of the GFP and the secretion of the vesicles are increased over time. From FIG. 7, it can be confirmed that since the vesicles has a size of 1 to 2 µm and contains the expressed GFP, the vesicles releases a green fluorescence signal. Further, from FIG. 8, it can be confirmed that the vesicles containing the GFP releases a red fluorescence signal of CellVue Claret, indicating that a phospholipid bilayer is present on the surface of the vesicles. From FIG. 9, it can be confirmed that the vesicles is derived from the cell and that a phospholipid bilayer having a thickness of about 9 nm is present on the surface of the generated vesicles, like the cell membrane. Further, from the observation that both the interior of the cell and the interior of the vesicles are darker than the surrounding area, it can be seen that the interior of the vesicles is rich in proteins, like the interior of the cell (osmium tetroxide is bonded to proteins to thus stabilize the proteins and increase the electron density at the part where the proteins are present, thereby creating light and shade when observed with a transmission electron microscope). Therefore, it can be seen that the excessive amount of the protein expressed by the delivery of the messenger RNA nanoparticles for GFP expression has a great influence on the secretion of vesicles from HeLa cells.

### 3. Production of vesicles by delivery of messenger RNA nanoparticles for RFP expression to HeLa cell

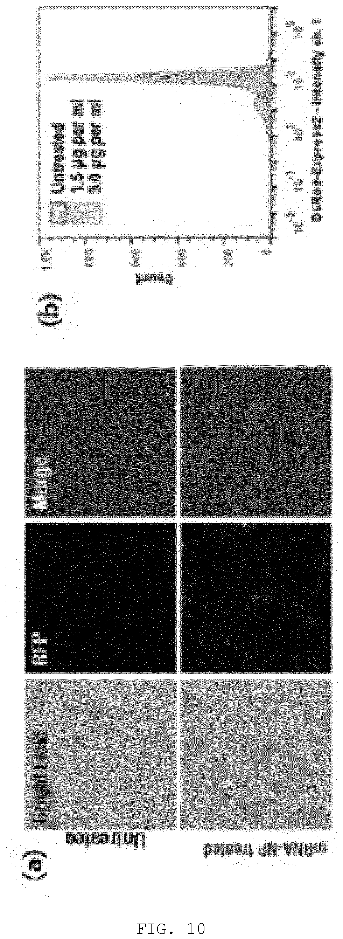

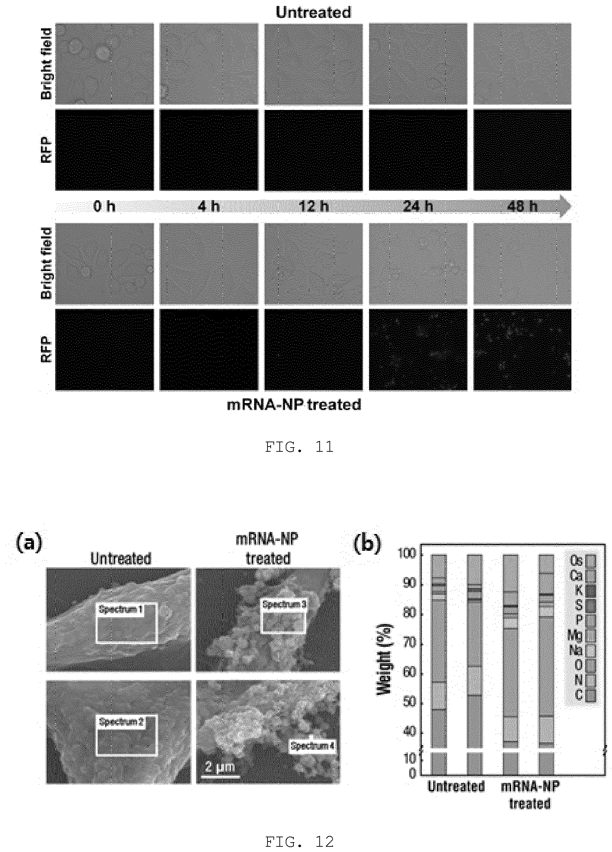

(1) The messenger RNA nanoparticles manufactured in item 2 of Example 1 were diluted in OPTI-MEM I and then mixed with a transfection reagent (TransIT-X2 Dynamic Delivery System) to form a complex (the ratio of messenger RNA nanoparticles: reagent: OPTI MEM I was 7 µg: 15 µl: 50 µl). The complex was added to a HeLa cell culture medium (20000 cells per cm² were dispensed), followed by reaction (treatment with 1.5 µg of the complex per 1 ml of the cell culture medium) . FIG. 10(a) shows the results of measurement using a fluorescence microscope 24 hours after the start of the above reaction. FIG. 10(b) shows the result of confirming RFP expression in cells by cytometry. Further, FIG. 11 shows the results of measurement using a fluorescence microscope 0, 4, 12, 24, and 48 hours after the start of the above reaction.
(2) From FIG. 10(a), it can be confirmed that a large amount of micro-sized vesicles (including RFP) is generated from mRNA-NP-treated cells treated with the messenger RNA nanoparticles for RFP expression, whereas the vesicles is not confirmed in untreated cells. From FIG. 10(b), it can be seen that a large amount of RFP was expressed in the cells treated with the messenger RNA nanoparticles through the fact that the intensity of red fluorescence was significantly higher in the cells treated with the messenger RNA nanoparticles for RFP expression than in the untreated cells. From FIG. 11, it can be confirmed that the secretion of the vesicles is clearly observed from the time point after 12 hours, and that the expression of the RFP and the secretion of the vesicles are then significantly increased over time. Accordingly, it can be seen that the excessive amount of the protein expressed by the delivery of the messenger RNA nanoparticles for RFP expression has a great influence on the secretion of the vesicles from HeLa cells.

### 4. Production of vesicles by delivery of messenger RNA nanoparticles for GFP expression to MDA-MB-231 cell

(1) The messenger RNA nanoparticles manufactured in item 1 of Example 1 were diluted in OPTI-MEM I and then mixed with a transfection reagent (TransIT-X2 Dynamic Delivery System) to form a complex (the ratio of messenger RNA nanoparticles: reagent: OPTI MEM I was 7 µg: 15 µl: 50 µl). The complex was added to a MDA-MB-231 cell culture medium (20000 cells per cm² were dispensed), followed by reaction (treatment with 1.5 µg of the complex per 1 ml of the cell culture medium). FIG. 12(a) shows the results of measurement of the cells stained with osmium tetroxide using a scanning electron microscope 24 hours after the start of the above reaction. FIG. 12(b) shows the analysis result of the weight% of the elements constituting the cells and the vesicles through SEM-based EDX (energy dispersive x-ray) analysis.
(2) From FIG. 12(a), it can be confirmed that a large amount of micro-sized vesicles is generated from mRNA-NP-treated cells treated with the messenger RNA nanoparticles for GFP expression, whereas the vesicles is not confirmed in untreated cells. As seen in FIG. 12(b), osmium is observed on the surface of the untreated cell and on the surface of the vesicles (osmium (Os) is observed due to the treatment using osmium tetroxide, which is used as a second fixative for observation of cells using a scanning electron microscope, and osmium has the property of binding to the head portion of the phospholipid of a cell membrane), indicating that both the cell and the vesicles are surrounded by a phospholipid bilayer. Phosphorus (P) that was observed serves to further demonstrate the presence of the phospholipid bilayer, which indicates that the vesicles is derived from the cell. Accordingly, it can be seen that the excessive amount of the protein expressed by the delivery of the messenger RNA nanoparticles for GFP expression has a great influence on the secretion of the vesicles from MDA-MB-231 cells.

### 5. Production of vesicles by delivery of messenger RNA nanoparticles for GFP or RFP expression to HDF cell

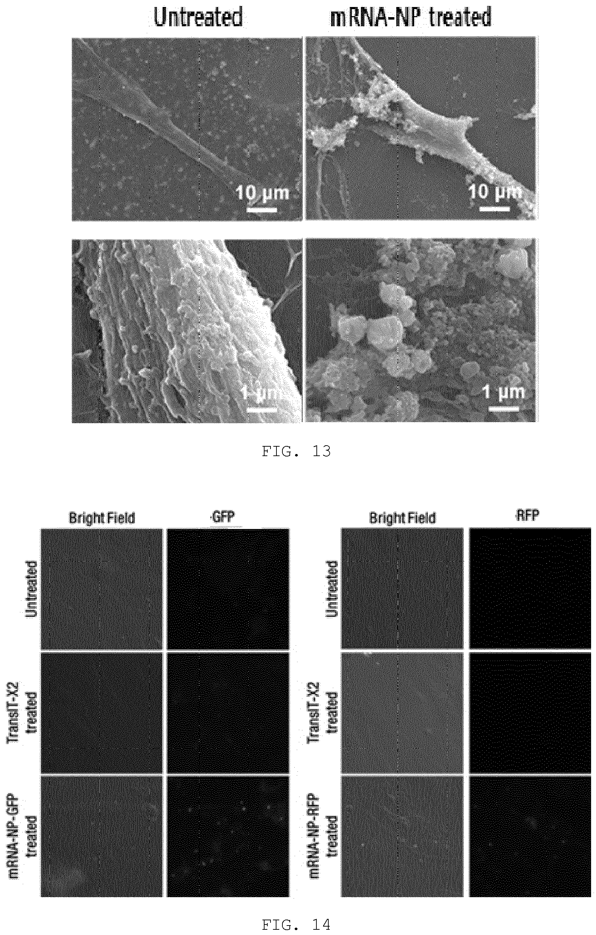

(1) The messenger RNA nanoparticles manufactured in item 1 of Example 1 were diluted in OPTI-MEM I and then mixed with a transfection reagent (TransIT-X2 Dynamic Delivery System) to form a complex (the ratio of messenger RNA nanoparticles: reagent: OPTI MEM I was 7 µg: 15 µl: 50 µl). The complex was added to a human dermal fibroblast (HDF) cell culture medium (20000 cells per cm² were dispensed), followed by reaction (treatment with 1.5 µg of the complex per 1 ml of the cell culture medium). FIG. 13 shows the results of measurement using a scanning electron microscope 24 hours after the start of the above reaction. Further, each of the messenger RNA nanoparticles for green fluorescent protein expression and the messenger RNA nanoparticles for red fluorescent protein expression manufactured in items 1 and 2 of Examples 1 was mixed with a transfection reagent (TransIT-X2 Dynamic Delivery system) to form complexes. The complexes and the transfection reagent containing no messenger RNA nanoparticles were added to the HDF cell culture medium (OPTI MEMI), followed by reaction. After 24 hours, the results of measurement using a fluorescence microscope are shown in FIG. 14.
(2) From FIG. 13, it can be confirmed that a large amount of micro-sized vesicles is generated from mRNA-NP-treated cells treated with the messenger RNA nanoparticles for GFP expression, whereas the vesicles is not confirmed in untreated cells. From FIG. 14, it can be confirmed that the green fluorescent protein and the red fluorescent protein are carried in the vesicles, which shows that the expressed protein is determined depending on the type of the messenger RNA nanoparticles and that the expressed protein is contained in the vesicles. It can be also confirmed that the transfection reagent, which was used to deliver the messenger RNA nanoparticles to the cells, does not affect the expression of the protein in the cells or the secretion of the vesicles. Accordingly, it can be seen that over-expressing the protein by the delivery of the messenger RNA nanoparticles has a great influence on the secretion of the vesicles containing the protein from HDF cells.

### 6. Evaluation of experimental results

From the above experimental results, it can be seen that the vesicles obtained by treating the cells with the messenger RNA nanoparticles for specific protein expression is derived from the cells and that the vesicles containing the target protein is capable of being mass-produced from various cell species.

### <Example 4> Confirmation of the possibility to adjust the amount of vesicles production

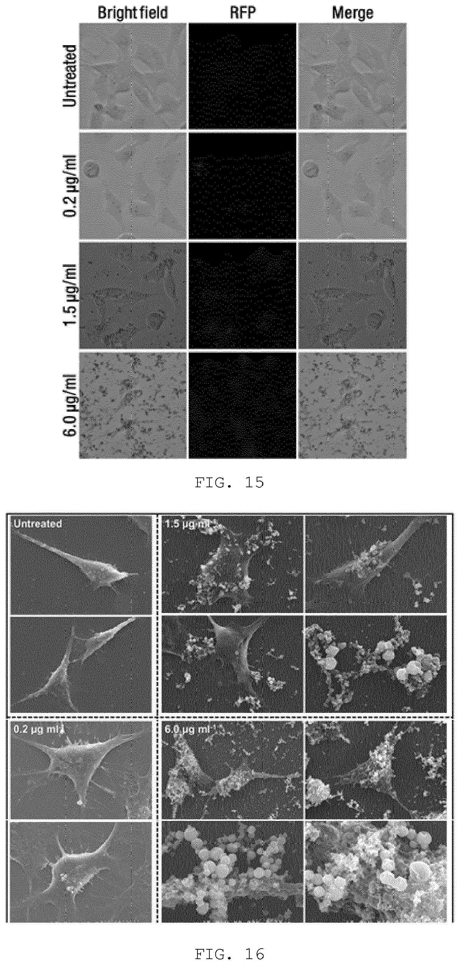

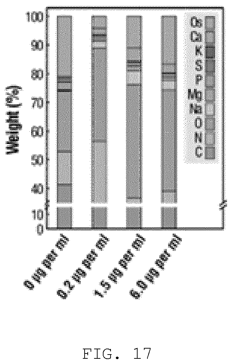

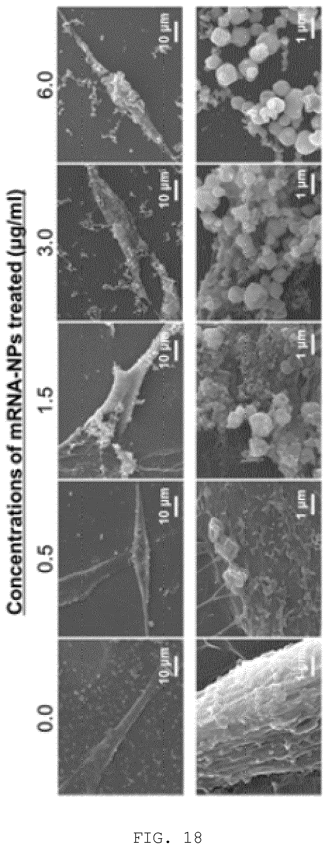

1. The messenger RNA nanoparticles manufactured in item 2 of Example 1 were diluted in OPTI-MEM I and then mixed with a transfection reagent (TransIT-X2 Dynamic Delivery System) to form a complex (the ratio of messenger RNA nanoparticles: reagent: OPTI MEM I was 7 µg: 15 µl: 50 µl). The complex was added to a HeLa cell culture medium (20000 cells per cm² were dispensed), followed by reaction (treatment with 0.2 µg, 1.5 µg, and 6 µg of the complex per 1 ml of the cell culture medium). FIGS. 15 and 16 show the results of measurement using a fluorescence microscope and measurement using a scanning electron microscope, respectively, 24 hours after the start of the above reaction. FIG. 17 shows the analysis result of the weight% of the elements constituting the cells and the vesicles through SEM-based EDX (energy dispersive x-ray) analysis.
2. Further, the messenger RNA nanoparticles manufactured in item 1 of Example 1 were diluted in OPTI-MEM I and then mixed with a transfection reagent (TransIT-X2 Dynamic Delivery System) to form a complex (the ratio of messenger RNA nanoparticles : reagent: OPTI MEM I was 7 µg: 15 µl: 50 µl). The complex was added to an HDF cell culture medium (20000 cells per cm² were dispensed), followed by reaction (treatment with 0.5 µg, 1.5 µg, 3 µg, and 6 µg of the complex per 1 ml of the cell culture medium). FIG. 18 shows the results of measurement using a scanning electron microscope 24 hours after the start of the above reaction.
3. From FIG. 15, it can be confirmed that the amount of the secreted vesicles is determined depending on the amount of the messenger RNA nanoparticles in the treatment and that the amount of the expressed protein, which is contained in the vesicles and is generated regardless of the amount of the messenger RNA nanoparticles, is constant. Further, from FIGS. 16 and 18, it can be confirmed that the vesicles containing the RFP was secreted while maintaining the cell shape in the treatment using 1.5 µg of the messenger RNA nanoparticles per 1 ml. However, in the treatment using the messenger RNA nanoparticles in an amount of four times (6.0 µg ml⁻¹), it can be confirmed that the overall shape of the cell is changed and that the cells are completely covered with the vesicles. Further, from FIG. 17, it can be confirmed that the cell has an element content similar to that of the parent cell regardless of the shape and size of the vesicles. Therefore, it can be seen that as the concentration of the messenger RNA nanoparticles is increased in the treatment, the size of the vesicles secreted from the cell is constant but the amount thereof is increased. When the treatment is performed using the messenger RNA nanoparticles at a predetermined concentration or higher, a large amount of the vesicles may be obtained in a short time, but most of the cell cytoplasm is released as vesicles, to thus cause damage to the cells. Accordingly, the concentration of the messenger RNA nanoparticles in the treatment may be adjusted so as to control the production of a large amount of the vesicles in a short time or the continuous production of the vesicles.

### <Example 5> Confirmation of the possibility of imparting functions to vesicles by reforming cell surface

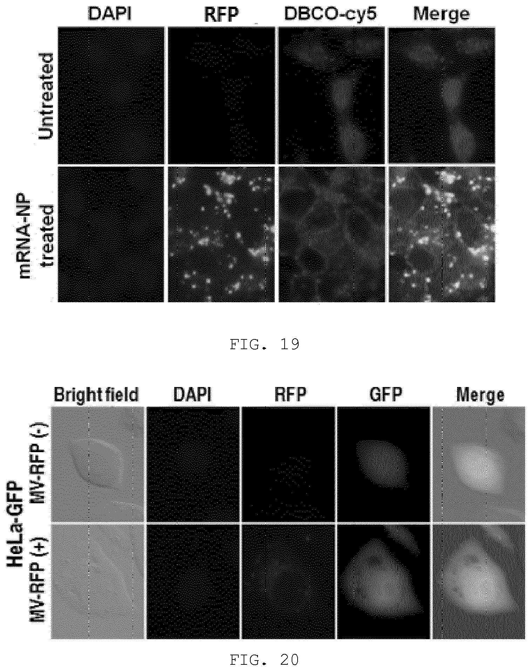

1. Ac3ManNAz (N-Azidoacetyl-D-mannosamine, triacetylated) was provided so as to be contained in a HeLa cell culture medium, followed by metabolic engineering, thus preparing a HeLa cell in which azide was expressed on the surface thereof. The messenger RNA nanoparticles manufactured in item 1 of Example 1 were diluted in OPTI-MEM I and then mixed with a transfection reagent (TransIT-X2 Dynamic Delivery System) to form a complex (the ratio of messenger RNA nanoparticles: reagent: OPTI MEM I was 7 µg: 15 µl: 50 µl). The complex was added to the HeLa cell culture medium (20000 cells per cm² were dispensed), followed by reaction (treatment with 1.5 µg of the complex per 1 ml of the cell culture medium). 24 hours after the start of the above reaction, the azide group that was present on the surface of the cell was reacted with DBCO-cy5 (Cyanine 5: red fluorescence substance) binding through click chemistry. Next, the nucleus of the cell was prepared so as to be stained with DAPI, and measurement was performed using a fluorescence microscope. The result is shown in FIG. 19.
2. From FIG. 19, it can be confirmed that in the case of cells treated with the messenger RNA nanoparticles, the vesicles containing the expressed GFP was formed and that DBCO-cy5 was labeled even on the surface of the vesicles, as on the surface of the cell. Accordingly, it can be seen that the vesicles is formed while the orientation of the cell membrane is maintained in the course of inducing the secretion of the vesicles containing the target protein by the delivery of the messenger RNA nanoparticles. Therefore, it can be seen that it is possible to impart new functions such as target recognition to the surface of the vesicles by reforming the surface of the cell using metabolic engineering.

### <Example 6> Confirmation of the possibility to use cell-derived vesicles as delivery system of target protein

1. The messenger RNA nanoparticles manufactured in item 2 of Example 1 were diluted in OPTI-MEM I and then mixed with a transfection reagent (TransIT-X2 Dynamic Delivery System) to form a complex (the ratio of messenger RNA nanoparticles: reagent: OPTI MEM I was 7 µg: 15 µl: 50 µl). The complex was added to a HeLa cell culture medium (20000 cells per cm² were dispensed), followed by reaction (treatment with 1.5 µg of the complex per 1 ml of the cell culture medium). After 24 hours, a solution containing the cells and MV were suspended in PBS, followed by centrifugation under a 300 RCF condition for 10 minutes. The resultant supernatant was subjected to centrifugation under a 2000 RCF condition for 10 minutes, and then the resultant supernatant was subjected to final centrifugation under a 10,000 RCF condition for 30 minutes, thus obtaining vesicles (MV-RFP) containing an RFP.
2. The HeLa-GFP cells transformed so as to express a green fluorescent protein in a cytoplasm were prepared. The use of the HeLa-GFP cells having a green color is intended to facilitate confirmation of the delivery of the cell-derived vesicles carrying a red fluorescent protein, which is a target protein.
3. The vesicles (MV-RFP) containing the RFP was added to the culture medium of the HeLa-GFP cells transformed such that the green fluorescent protein was uniformly contained in the cytoplasm, followed by reaction. After the nucleus of the cell was stained with DAPI, measurement was performed using a fluorescence microscope. The result is shown in FIG. 20.
4. From FIG. 20, it can be confirmed that the target protein RFP, delivered using the vesicles, was successfully delivered to the interior of the HeLa-GFP cell containing the vesicles, indicating that the vesicles is capable of being utilized as a drug delivery system.

Although the applicant has described preferred embodiments of the present invention, it is to be understood that such embodiments are merely exemplary embodiments of the technical idea of the present invention, and that they are intended to cover various changes or modifications included within the spirit and scope of the invention.

## Claims

1. A method of manufacturing vesicles, the method comprising:
a delivery step of delivering RNA nanoparticles for vesicles production to a cell; and
a culturing step of culturing the cell to which the RNA nanoparticles for vesicles production are delivered after the delivery step, thus generating the vesicles containing a target protein.

2. The method of claim 1, wherein the RNA nanoparticles for vesicles production include repeated messenger RNA for target protein expression, so that the protein is locally over-expressed in the cell in the culturing step to thus be excreted through the vesicles to an outside of the cell, which enables the vesicles containing the target protein to be produced.

3. The method of claim 2, wherein an amount of the RNA nanoparticles for vesicles production delivered to the cell in a delivery step is adjusted, thus adjusting a generation amount and a manufacturing time of the vesicles.

4. The method of claim 2, wherein the RNA nanoparticles for vesicles production have a spherical shape and are 50 to 200 nm in diameter.

5. The method of claim 2, further comprising:
a surface-reforming step of reforming a surface of the cell before a delivery step,
wherein, since the vesicles generated during the culturing step has surface properties of the cell, when the cell having the surface reformed during the surface-reforming step is used in the delivery step, the vesicles carrying the target protein and having a surface property of a specific function is produced in the culturing step.

6. The method of claim 2, wherein a delivery step includes a process using a liposome or a positively charged polymer, a delivery process using the liposome includes adding the RNA nanoparticles to a lipid-based-complex-forming solution and then forming a complex in order to treat the cell, and a process using the positively charged polymer includes dispensing the cell on a culture dish, adding a growth medium thereto to culture the cell for a predetermined time, and adding a complex obtained by mixing the RNA nanoparticles and the positively charged polymer to the growth medium.

7. The method of claim 2, further comprising:
a separation step of separating the vesicles generated in the culturing step.

8. The method of claim 7, wherein in the separation step, the vesicles containing a target protein is separated from a cell culture medium using centrifugation.


<110>     University of Seoul Industry Cooperation Foundation

<120>     Method of manufacturing vesicles by delivery of RNA nanoparticles
          and vesicles manufactured using the same

<130>     2019/0807

<150>     KR 10-2019-0005784
<151>     2019-01-16

<160>     5

<170>     KoPatentIn 3.0

<210>     1
<211>     19
<212>     DNA
<213>     Artificial Sequence

<220>
<223>     promoter region of plasmid DNA for RNA polymerase


<400>     1
taatacgact cactatagg                                                    19


<210>     2
<211>     10
<212>     DNA
<213>     Artificial Sequence

<220>
<223>     region of plasmid DNA for binding ribosome


<400>     2
gccaccatgg                                                              10


<210>     3
<211>     720
<212>     DNA
<213>     Artificial Sequence

<220>
<223>     region of plasmid DNA for GFP generation


<400>     3
atggtgagca agggcgagga gctgttcacc ggggtggtgc ccatcctggt cgagctggac        60

ggcgacgtaa acggccacaa gttcagcgtg tccggcgagg gcgagggcga tgccacctac       120

ggcaagctga ccctgaagtt catctgcacc accggcaagc tgcccgtgcc ctggcccacc       180

ctcgtgacca ccctgaccta cggcgtgcag tgcttcagcc gctaccccga ccacatgaag       240

cagcacgact tcttcaagtc cgccatgccc gaaggctacg tccaggagcg caccatcttc       300

ttcaaggacg acggcaacta caagacccgc gccgaggtga agttcgaggg cgacaccctg       360

gtgaaccgca tcgagctgaa gggcatcgac ttcaaggagg acggcaacat cctggggcac       420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaagtaa 720

720

<210> 4
<211> 574
<212> DNA
<213> Artificial Sequence

<220>
<223> region of plasmid DNA for binding ribosome

<400> 4
cccctctccc tccccccccc ctaacgttac tggccgaagc cgcttggaat aaggccggtg 60

tgcgtttgtc tatatgttat tttccaccat attgccgtct tttggcaatg tgagggcccg 120

gaaacctggc cctgtcttct tgacgagcat tcctaggggt ctttcccctc tcgccaaagg 180

aatgcaaggt ctgttgaatg tcgtgaagga agcagttcct ctggaagctt cttgaagaca 240

aacaacgtct gtagcgaccc tttgcaggca gcggaacccc ccacctggca acaggtgcct 300

ctgcggccaa aagccacgtg tataagatac acctgcaaag gcggcacaac cccagtgcca 360

cgttgtgagt tggatagttg tggaaagagt caaatggctc tcctcaagcg tattcaacaa 420

ggggctgaag gatgcccaga aggtacccca ttgtatggga tctgatctgg ggcctcggtg 480

cacatgcttt acatgtgttt agtcgaggtt aaaaaaacgt ctaggccccc cgaaccacgg 540

ggacgtggtt ttcctttgaa aaacacgatg ataa 574

<210> 5
<211> 678
<212> DNA
<213> Artificial Sequence

<220>
<223> region of plasmid DNA for RFP generation

<400> 5
   atggatagca ctgagaacgt catcaagccc ttcatgcgct tcaaggtgca catggagggc 60
   tccgtgaacg gccacgagtt cgagatcgag ggcgagggcg agggcaagcc ctacgagggc 120
   acccagaccg ccaagctgca ggtgaccaag ggcggccccc tgcccttcgc ctgggacatc 180
   ctgtcccccc agttccagta cggctccaag gtgtacgtga agcaccccgc cgacatcccc 240
   gactacaaga agctgtcctt ccccgagggc ttcaagtggg agcgcgtgat gaacttcgag 300
   gacggcggcg tggtgaccgt gacccaggac tcctccctgc aggacggcac cttcatctac 360
   cacgtgaagt tcatcggcgt gaacttcccc tccgacggcc ccgtaatgca gaagaagact 420
   ctgggctggg agccctccac cgagcgcctg tacccccgcg acggcgtgct gaagggcgag 480
   atccacaagg cgctgaagct gaagggcggc ggccactacc tggtggagtt caagtcaatc 540
   tacatggcca agaagcccgt gaagctgccc ggctactact acgtggactc caagctggac 600
   atcacctccc acaacgagga ctacaccgtg gtggagcagt acgagcgcgc cgaggcccgc 660
   caccacctgt tccagtag 678